# EUROPEAN PATENT SPECIFICATION

(11) **EP 4 011 348 B1**
(45) Date of publication and mention of the grant of the patent: **14.10.2026**
(21) Application number: 20851565.0
(22) Date of filing: 06.08.2020
(51) Int. Cl.: A61H 3/00, A61F 5/02, A61F 5/01

(54) **ORTHOTIC**
ORTHESE
ORTHÉTIQUE

(30) Priority: 09.08.2019 JP 2019147629
(43) Date of publication of application: 15.06.2022
(73) Proprietor: Toray Industries, Inc., Tokyo 103-8666 (JP)
(72) Inventor: YAMADA, Satoshi, Otsu-shi, Shiga 520-2141 (JP); CHEN, Yao, Otsu-shi, Shiga 520-2141 (JP); SUZUKI, Hidetoshi, Otsu-shi, Shiga 520-2141 (JP)
(74) Representative: Mewburn Ellis LLP
(86) International application number: PCT/JP2020/030225
(87) International publication number: WO 2021/029325

(56) References cited:
- EP-A1- 2 316 288
- EP-A1- 2 526 792
- JP-A- 2017 186 726
- JP-A- 2018 011 903
- JP-A- 2019 081 986
- US-A1- 2019 110 916

## Description

### TECHNICAL FIELD

The present invention relates to an orthotic.

### BACKGROUND ART

Conventionally, various orthotics have been proposed for preventing deformation such as equinus foot, contracture, and scoliosis, function improvement, and daily operation improvement for a person who cannot freely move muscles and joints due to functional deterioration or loss of the body due to disorders such as stroke hemiplegia and infantile paralysis, or other disorders. The orthotics are mainly hard holding tools such as metal and plastic, and are excellent in holding force and orthotic force to an affected part.

In addition, an orthotic using a flexible material has been proposed, and for example, Patent Document 1 has disclosed a foot orthotic capable of lifting the toe upward without blocking the operation of the foot. Specifically, the technique includes a first attachment to be attached to a foot of a person, a second attachment to be attached to a thigh of the person, and an elastic material such as a rubber band to be attached so as to be stretched between the first attachment and the second attachment. When the elastic material is attached so as to extend, an elastic force of the elastic material acts to bring the first attachment and the second attachment close to each other. As a result, the foot is elastically urged toward the front side of the lower leg, and the toe is lifted upward. In addition, there has been disclosed a technique in which the urging force allows operation of the foot in a certain degree, and in a case where the function of lowering the toe is not impaired, the toe can be lowered by itself against the urging force. Patent Documents 2 to 5 disclose further wearables.

### PRIOR ART DOCUMENT;PATENT DOCUMENT

| | |
|---|---|
| Patent Document 1: | JP 09-313553 |
| Patent Document 2: | JP 2017-210970 A |
| Patent Document 3: | EP 2316288 A1 |
| Patent Document 4: | US 2019/0110916 A1 |
| Patent Document 5: | EP 2526792 A1 |

### SUMMARY OF THE INVENTION

### PROBLEMS TO BE SOLVED BY THE INVENTION

However, a fixed type orthotic using a hard or semi-hard material such as metal or plastic has had many problems of physical and mental pain of a patient, such as discomfort caused by contact with the skin when the orthotic is worn, pain caused by hitting of a hard part in body operation, poor appearance, and the like, as well as has been pointed out from caregivers to be poor detachability and difficulty in eating.

In addition, the orthosis described in Patent Document 1 works by bringing the two attachments close to each other with the elastic material, and is problematic in that it takes time and effort to wear the two attachments and to mount the elastic material. In addition, the elastic material is composed of an elastically stretchable material represented by a rubber band, and the urging force thereof allows operation of the foot in a certain degree, which is problematic in that the fixing of the orthotic site is insufficient and a necessary orthotic force cannot be obtained.

An object of the present invention is to provide an orthotic that can improve the problems of the prior art, can achieve both an orthotic force necessary for improvement of deformation and function of a body and the trackability to friction, compression and the like between an orthotic site and the orthotic, which occur during operation, is easy to wear, does not impair an appearance in wearing, maintains an appropriate holding force and orthotic force for a long period of time, and thus can be continuously used.

### SOLUTIONS TO THE PROBLEMS

In order to solve such a problem, the present invention is defined in claim 1.

In some embodiments, the orthotic further comprises a region A that covers a periphery of the orthotic site, wherein the region A includes at least one or more of structures having elastic properties.

In some embodiments of the orthotic, an extension recovery ratio of the woven fabric structure used in the region B after 50 cycle extension under a load of 100 N/5 cm is 80% to 100% in a warp direction and a weft direction or any one of these directions.

In some embodiments of the orthotic, the region B1 and/or the region B2 comprise a mechanism capable of adjusting fastening force during wearing.

In some embodiments, the orthotic further comprises a mechanism that is openable and closable during wearing.

In some embodiments of the orthotic, the region A and the region B are integrally formed by being connected to each other.

In some embodiments, the orthotic is used as an orthosis for rehabilitation. In some embodiments, the orthotic is an upper limb orthosis. In some embodiments, the orthotic is a trunk orthosis. In some embodiments, the orthotic is a lower limb orthosis.

### EFFECTS OF THE INVENTION

The present invention can provide an orthotic that achieves both a holding force and an orthotic force necessary for improvement of deformation and function of a body and trackability to friction, fluctuation, compression and the like occurring during operation between an orthotic site and the orthotic, is easy to wear, does not impair appearance during wearing, and in addition, maintains an appropriate holding force and orthotic force for a long period of time, and can be continuously used.

The orthotic according to the present invention can be preferably used for orthoses such as an upper limb orthosis, a trunk orthosis, and a lower limb orthosis for treatment and rehabilitation for the purpose of preventing deformation of the body, improving the function, and improving the operation of daily life; however, the application range is not limited thereto.

### BRIEF DESCRIPTION OF THE DRAWINGS

Fig. 1 shows a conceptual view of an embodiment of a region B of the present invention.
Fig. 2 shows a side view of an orthotic prepared in Example 1.

### EMBODIMENTS OF THE INVENTION

In the present invention, for the purpose of preventing deformation of the body, improving the function, and improving the operation of daily life, the orthotic is an orthosis for exhibiting an orthotic force for guiding the body state to a normal state, and can be used for treatment, rehabilitation, and the like. For example, the orthotic can be used as orthoses such as an upper limb orthosis, a trunk orthosis, or a lower limb orthosis to be attached to an upper limb, a trunk, or a lower limb. Then, the wearer can live or be active in a more comfortable state or more continuously by wearing an orthotic and performing operation of daily life while exhibiting an orthotic force, or by performing rehabilitation for prevention of deformation and function improvement of the body to guide the body state to a more normal state.

The orthotic according to the present invention includes a region B that exhibits an orthotic force when the orthotic is worn. In the orthotic of a preferred aspect, the orthotic includes a region A that covers the periphery of the orthotic site and a region B that exhibits an orthotic force when the orthotic is worn.

The region A covering the periphery of the orthotic site is adjacent to the region B, and has a function of being comfortably worn as an orthotic.

At least one or more structures having elastic properties are preferably used for the region A. This structure is not particularly limited, and a knitted fabric material is preferably used for imparting elastic properties. As the knitted fabric material, there are a warp knitted material and a circular knitted material, and any one may be adopted as long as the elastic properties are good.

In addition, the yarn to be used may be either a short fiber or a filament. The material of the yarn is not limited to natural fibers and synthetic fibers, and various fibers can be used. Specifically, synthetic fibers such as polyester and polyamide, cellulose fibers such as rayon and cotton, and natural fibers such as wool and silk can be appropriately used. The above materials may be used in combination of interknitting, inserting, aligning, and the like. In addition, in order to impart elastic properties, an elastic fiber that is a stretchable material is preferably combined with the above material. The elastic fibers to be combined include polyurethane elastic fibers, polyester elastomer fibers, and PTT-based composite textured yarns (side-by-side bimetal yarns including polytrimethylene terephthalate as a main component).

The region A is not particularly limited as long as it can cover the periphery of the orthotic site, and the physical stimulation to the orthotic site can be alleviated by providing the region A in the periphery of the orthotic site (the orthotic site and/or the periphery thereof) between the body side of the orthotic site and at least a part of the region B. For example, when the orthotic is for a lower limb, the orthotic site can be preferably covered inside at least a part of the regions B1 to B3 in the form of a sock. In the case of the upper limb, the orthotic site can be preferably covered inside at least a part of the regions B1 to B3 in the form of a long glove. In the case of the trunk, the orthotic site can be preferably covered inside at least a part of the regions B1 to B3 in the form of a shirt.

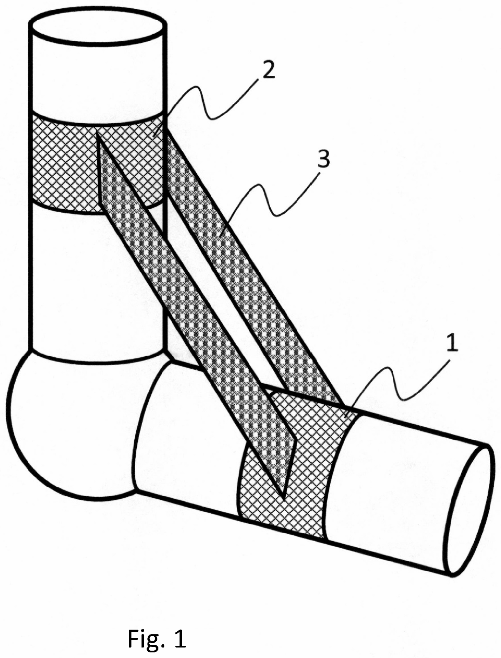

The region B has a function of exhibiting an orthotic force on the orthotic site when the orthotic is worn. The region B includes a region B1 that covers an orthotic site, a region B2 that is a support region for applying an orthotic force for the orthotic site, and a region B3 that connects the region B1 and the region B2. Fig. 1 shows a conceptual view of an embodiment of a region B of the present invention. A region A in Fig. 1 is not illustrated.

Specifically, the region B1 (1) has a function of preventing the orthotic site from moving in a direction other than the desired orthotic direction by covering and fixing the orthotic site.

The region B2 (2) has a function as a fulcrum for maintaining the orthotic force without reducing the orthotic force such as deviation, tear, or excessive extension for the desired orthotic force.

The region B3 (3) has a function of connecting the region B1 (1) and the region B2 (2) and setting a desired orthotic force by, for example, adjusting a connection length in order to apply the orthotic force. The method of connecting the region B1 and the region B2 can be appropriately selected as long as a desired orthotic force is satisfied. Specifically, there is used sewing with a sewing machine or the like, a hook-and-loop fastener, a cloth tape, a snap button, or the like.

The above orthotic site is a site that undergoes action for promoting correction to an original normal position or for improving the function regarding body deformation such as equinus foot, contracture, and scoliosis and functional deterioration such as walking.

Specifically, the above orthotic site may be a site that causes symptoms, that is, functional deterioration such as body deformation and walking, or may be a site that can indirectly improve symptoms by undergoing action although the site does not cause symptoms.

The structure of the region B includes a woven fabric structure from the viewpoint of a holding force for exhibiting an orthotic force and dimensional stability.

In addition, the woven fabric structure used for the region B has elastic properties, and the extension ratio when a load is 100 N/5 cm, that is, the extension ratio for extension under a load of 100 N/5 cm is 1.0% to 20%, more preferably 2.0% to 20%, and still more preferably 3.0% to 20% in a warp direction and a weft direction or any one of these directions. The extension ratio is set within the above range not to excessively hold the orthotic site. In addition, preferably, the above woven fabric structure is appropriately stretched to reduce the friction between the orthotic site and the orthotic, and to provide improved trackability to fluctuation of the orthotic site during operation, and to compression on the site covered by the region B, which occurs when the orthotic force acts, and as a result, pain and discomfort in wearing are easily suppressed. In addition, preferably, the woven fabric structure receives the force applied by the wearer during operation, whereby it is easy to move muscles, and thus it is effective for function recovery and daily operation improvement.

The weave structure of the woven fabric structure requires that the range defined in the present invention is satisfied; however, a structure such as plain weave is used.

In addition, the yarn to be used may be either a short fiber or a filament as long as the range defined in the present invention is satisfied. The material of the yarn is not limited to natural fibers, synthetic fibers, and the like as long as the range defined in the present invention is satisfied, and various fibers can be used.

Specifically, there can be appropriately used synthetic fibers such as polyester fibers and polyamide fibers, cellulose fibers such as rayon and cotton, and natural fibers such as wool and silk. The above materials may be used in combination of interknitting inserting, aligning, and the like.

In addition, in order to impart elastic properties, an elastic fiber that is a stretchable material is combined with the above material. The elastic fibers to be combined include polyester elastomer fibers, and may also include polyurethane elastic fibers, PTT-based composite textured yarns (side-by-side bimetal yarns including polytrimethylene terephthalate as a main component) and the like, and polyester elastomer fibers are used from the viewpoint of easily obtaining dimensional stability and elastic properties with high elasticity.

The fineness of the fiber used for the region B is preferably 30 decitex to 1500 decitex, and more preferably 50 decitex to 1000 decitex. When the above elastic fibers and other fibers (inelastic fibers) are used, the fineness of the elastic fibers is preferably 50 decitex to 1500 decitex, and more preferably 156 decitex to 1000 decitex. The fineness of the inelastic fibers as other fibers is preferably 30 decitex to 1000 decitex, and more preferably 50 decitex to 500 decitex.

Elastic fibers are used as a part of the yarns used as described above. Specifically, a configuration is adopted such that an elastic fiber is used for any one of the warp and the weft or at least a part of the both, thereby allowing appropriate adjustment of: the dimensional stability of the woven fabric; the elastic properties of the elastic fiber and a good recoverability after extension, the holding force or dimensional stability to exhibit the orthotic force in the orthotic site; and the trackability to the operation.

In the above case, the use ratio of the elastic fibers to be used is appropriately determined within a range in which desired characteristics are obtained, and is preferably about 30 wt% to 70 wt% in the warp or weft, or in the entire woven fabric.

In addition, when other fibers (inelastic yarns in claim 1) that are fibers other than elastic fibers are used in the woven fabric structure, the extension ratio can be adjusted by appropriately adjusting the crimp ratio. Since, in claim 1, an inelastic fiber is used for either the warp or the weft and the crimp ratio thereof is 10% or more and 30% or less, desired extensibility can be obtained.

In addition, when elastic fibers and inelastic fibers are used for either the warp or the weft, the crimp ratio of the inelastic fibers is preferably 10% or more and 30% or less. As a result, when the elastic fibers are extended, the inelastic fibers follow the crimp ratio, and excessive extension by the elastic fibers can be stopped.

The crimp ratio of the elastic fiber in this case is also appropriately set according to desired characteristics, and is 0% or more and 5% or less.

The weave density of the woven fabric structure used in the present invention is preferably 20 yarns/inch (2.54 cm) to 150 yarns/inch (2.54 cm), more preferably 30 yarns/inch (2.54 cm) to 100 yarns/inch (2.54 cm) in warp density and weft density, respectively.

The extension recovery ratio of the woven fabric structure used for the region B after 50 cycle extension at a load of 100 N/5 cm is preferably 80% to 100%, more preferably 85% to 100%, and still more preferably 90% to 100% in a warp direction and a weft direction or any of these directions. Preferably, the extension recovery ratio after 50 cycle extension is set within the above range and the force to return to the original orthotic position moderately acts on the force applied by the wearer during operation, whereby an appropriate orthotic position is maintained, which is effective for function recovery and daily operation improvement. In addition, the appropriate holding force and the orthotic force are maintained for a long period of time, allowing the orthotic of the present invention to be continuously used, which are more effective for function recovery and daily operation improvement.

In the orthotic of the present invention, the spring constant of the structure used in the region B1 and the region B3 is 10 N/mm or more and 100 N/mm or less, preferably 20 N/mm or more and 100 N/mm or less, and more preferably 30 N/mm or more and 100 N/mm or less in a warp direction and a weft direction, or any one of these directions. The spring constant is set within the above range, appropriately correcting the orthotic site, providing better trackability to friction, compression, and the like with the orthotic, and easily suppressing pain and discomfort during wearing.

In order to obtain a woven fabric structure having the above spring constant, elastic fibers are used as a part of the yarns to be used. Specifically, a configuration is adopted such that an elastic fiber is used for any one of the warp and the weft or at least a part of the both, providing the elastic properties of the elastic fiber and good recoverability after extension. The ratio of using the elastic fiber is adjusted, thereby allowing providing a desired spring constant. The weave structure is plain weave in claim 1. The elastic fibers to be combined include polyester elastomer fibers, and may also include polyurethane elastic fibers, PTT-based composite textured yarns (side-by-side bimetal yarns including polytrimethylene terephthalate as a main component) and the like, and polyester elastomer fibers are used from the viewpoint of easily obtaining elastic properties with high elasticity.

A woven fabric structure in the present invention is a plain woven fabric composed of inelastic fibers with one of the warp and the weft having a crimp ratio of 10% or more and 30% or less and elastic fibers with at least a part of the other having a crimp ratio of 0% or more and 5% or less. In addition, the weave density is preferably 30 yarns/inch (2.54 cm) to 100 yarns/inch (2.54 cm) in warp density and weft density.

Furthermore, the fineness is preferably 50 decitex to 500 decitex for inelastic fibers and 156 decitex to 1000 decitex for elastic fibers.

The orthotic of the present invention preferably includes a mechanism capable of adjusting the fastening force during wearing in the region B1 and/or the region B2. The fastening force is optionally adjusted, whereby the region B1 and/or the region B2 does not deviate from a predetermined position, come off, or the like when the orthotic of the present invention is worn and operated, the orthotic portion can be stably covered and gripped in the region B1, and the regions B1 and B3 preferably act as a support exhibiting the orthotic force in the region B2. The above adjustment mechanism is not particularly limited, and can be appropriately selected according to the application, such as a belt, a hook, a cloth tape, a button, or a buckle.

The orthotic of the present invention preferably includes a mechanism that can be opened and closed during wearing. The mechanism that can be opened and closed during wearing is a mechanism that can be opened and closed to expand an opening when the orthotic of the present invention is worn and removed, or can open and close a portion that is not normally opened during wearing to facilitate wearing and removing. A portion with the openable and closable mechanism provided is not particularly limited as long as it is a portion where the orthotic function is not impaired and wearing and removing are facilitated, and the mechanism can be provided in any one of the region A (when the region A is used) and the regions B1 to B3 or in the connecting portion thereof. The mechanism that can be opened and closed during wearing is included, whereby a wearer with strong deformation such as equinus foot and contracture, an aged wearer, and the like can wear and remove the orthotic by themselves, the wearing and removing work is reduced for the caregiver, and use of the orthotic is not avoided and can be continuous. When the orthotic of the present invention can be continuously used, this case is more effective for function recovery and daily operation improvement. The openable and closable mechanism is not particularly limited, and can be appropriately selected according to the application, for example, a fastener such as a zip fastener or a hook-and-loop fastener, or a button such as a cloth tape, a snap button, or a tack button.

For the orthotic of the present invention, only the region B may be used as long as desired characteristics such as orthotic force and wearing-and-removing properties are satisfied in the region B. In addition, the region A and region B may be used as separate members or may be partially connected to each other as long as desired characteristics such as orthotic force and wearing-and-removing properties are satisfied. Of these, more preferably, the region A and the region B are connected and integrally formed. The region A and the region B are connected and integrally formed, whereby it does not take time and effort during wearing, and wearing can be performed in the same manner as general clothing. As a result, the burden on the wearer and the caregiver can be reduced, and the orthotic of the present invention can be continuously used, which is more effective for function recovery and daily operation improvement. The connection method is not particularly limited, and can be appropriately selected according to the application such as sewing and adhesion.

The orthotic thus obtained has an orthotic force necessary for deformation and functional improvement of the body, has trackability to friction, compression, and the like occurring during operation between the orthotic site and the orthotic, can be continuously used, is easy to wear, and does not impair appearance during wearing, and thus can be preferably used for orthoses such as upper limb orthosis, trunk orthosis, and lower limb orthosis. The application of wearing thereof is not only preferable as an orthosis for daily life, but also extremely useful as an orthosis for rehabilitation.

### EXAMPLES

Hereinafter, examples of the present invention will be described together with comparative examples.

Methods of measuring various characteristics in the present embodiment are as follows.

### (1) Fineness

The fineness was measured in accordance with JIS L 1013: 2010 8.3.1 fineness based on corrected mass (method A).

### (2) Extension ratio under load of 100 N/5 cm

The extension ratio of the woven fabric structure used in the region B was measured in accordance with the strip method (method A) of JIS L 1096: 2010 8.14.1 JIS method. That is, 5 pieces of samples each having a size of 5 cm × 30 cm were taken in the warp and the weft directions, respectively. Using a constant-speed extension-type tensile tester with an automatic recording device, the grip interval was set to 20 cm, and the slack and tension of the sample were removed and then the sample was fixed to the grip. The sample was extended to 100 N at a tensile speed of 200 mm/min, the grip interval was then measured, the extension ratio LA (%) was determined by the following formula, and the average value for 5 pieces of samples was taken. $Extension ratio LA \left(%\right) = \left[\left(L1-L\right)/L\right] \times 100$
L: grip interval (mm)
L1: grip interval at an extension to 100 N (mm)

### (3) Extension recovery ratio after 50 cycle extension at load of 100 N/5 cm

The extension recovery ratio of the woven fabric structure used in the region B was measured with reference to the strip method (method A) of JIS method, JIS L 1096: 2010 8.15.1. That is, 5 pieces of samples each having a size of 5 cm × 30 cm were taken in the warp and the weft directions, respectively. Using a constant-speed extension-type tensile tester with an automatic recording device, the sample was marked with a grip interval of 200mm (Lb), and the slack and tension of the sample were removed and then the sample was fixed to the grip. The sample was extended to a value of an extension ratio (LA in the previous section) separately obtained at a tensile speed of 200 mm/min, left for 1 minute, then returned to the original position at the same speed, and left for 3 minutes. This operation was repeated 50 cycles, then the load was removed, and the sample was left for 3 minutes. Then, the slack and tension of the sample were removed, and the length (Lb1) between the marks was measured. The extension recovery ratio LB (%) was determined by the following formula, and the average value for 5 pieces of samples was taken. 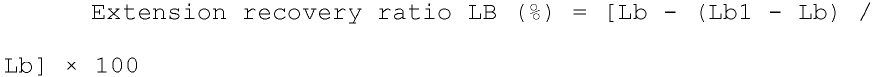 Extension recovery ratio LB (%) = [Lb - (Lb1 - Lb) / Lb] × 100

### (4) Spring constant

Referring to JIS-K-6400-2: 2012E method, the center of a pressurizing jig of an elliptic plate with R30 (minor axis 250 mm × major axis φ 300 mm) was aligned with the center position of the fabric, the contacting position was set to the initial position (0 N), and the test was started, with using a static load deflection tester for sheets (FGS-TV series, 2-axis manual motion, manufactured by Nidec-Shimpo Corporation). Pressurization was performed up to a load of 200 N or more at a pressurization rate of 270 mm/min, and the inclination of the tangent line of the graph between the load at 200 N load and the sinking amount [mm] was measured for each of 5 pieces of samples, and the average thereof was calculated.

### (5) Wearing evaluation

The produced orthotic was worn on one foot requiring correction, and in a state of walking for 1 hour, three items of orthotic force, compression feeling, and rubbing were evaluated. Evaluation was performed by sensory evaluation of wearers, and evaluation was performed in three stages of 5, 3, and 1.

### A. Orthotic force

5: the orthotic state was maintained, and the region B was slightly tracked when force was applied.
3: the orthotic state was strongly maintained, and the region B unchanged when force was applied.
1: retention in the orthotic state was weak, and the region B was tracked when force was applied.

### B. Compression feeling

5: there was a feeling of strangeness in a portion covered by the region B during walking; however, it was not noticeable.
3: there was a feeling of compression in a portion covered by the region B during walking; however, there was no pain.
1: there was a strong feeling of compression in a portion covered by the region B, and there was pain.

### C. Rubbing

5: there was a no feeling of pain around the region B during walking.
3: there was a no feeling of pain around the region B during walking; however, there was a feeling of strangeness like rubbing.
1: there was a feeling of pain by rubbing with the skin around the region B during walking.

### (6) Woven density

Measurement was performed in accordance with JIS L 1096: 2010 8.6.1 (method A). The sample was placed on a flat table, unnatural wrinkles and tension were removed, the number of warps and wefts existing at an interval of 1 inch (2.54 cm) was counted at 5 different locations, and the average value of each number was calculated.

### (7) Crimp ratio

The crimp ratios for the warp and weft taken from the woven fabric were measured in accordance with the method of JIS L1096 8.7b. The measurement was performed for 20 yarns, and the average value thereof was taken.

### [Example 1]

In the region A, there was used a tricot fabric obtained by interweaving two types of fibers, a polyester fiber of 54 decitex and a polyurethane fiber of 156 decitex as elastic fibers. In the region B, using 167 decitex polyester fibers of inelastic yarns as the warp and 480 decitex polyester elastomer "Hytrel" (registered trademark) monofilament of elastic yarns as the weft, a plain woven fabric having a warp density of 39 yarns/inch (2.54 cm) and a weft density of 43 yarns/inch (2.54 cm) was produced. The obtained woven fabric was heat-treated at a temperature of 180°C for 2 minutes to provide a woven fabric structure having a warp density of 45 yarns/inch (2.54 cm) and a weft density of 46 yarns/inch (2.54 cm). The crimp ratios of the warp and the weft were 20% and 3%, respectively.

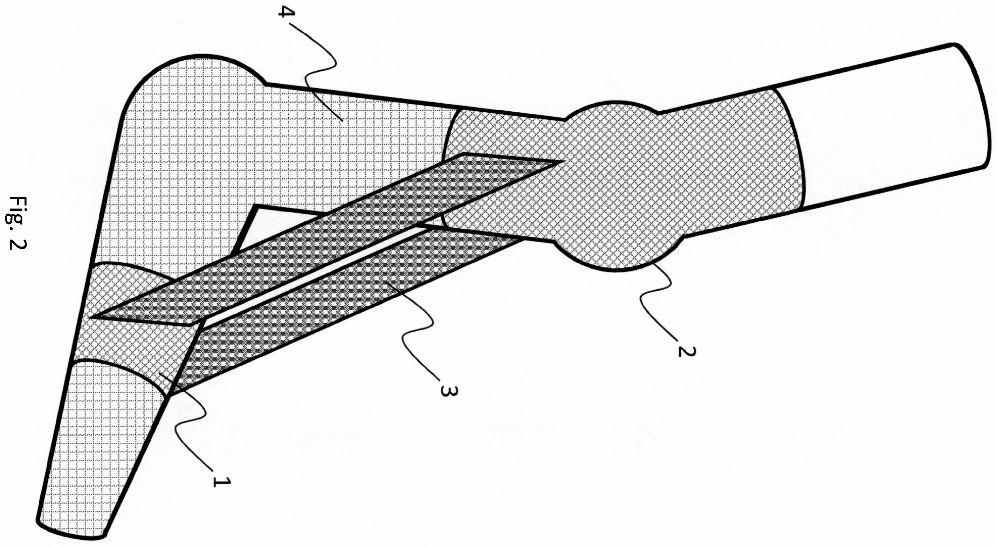

The obtained woven fabric structure had extension ratios of 5% for warp and 15% for weft under a load of 100 N/5 cm, extension recovery ratio of 85% for warp and 95% for weft, and a spring constant of 50 N/mm. A sock type orthotic was produced by using the fabrics of the region A and the region B. Fig. 2 is a side view of the orthotic. The region A (4) had a sock shape covering from a foot to a knee of one foot. The region B1 (1) was covered over the periphery of the metatarsal of the foot such that the width direction of the foot and the weft direction of the obtained fabric structure were aligned, and the region B2 (2) was covered over the periphery of the knee including the popliteal fossa such that the periphery direction of the knee and the weft direction of the obtained fabric structure were aligned. The region B3 (3) was connected from both side surfaces of the region B2 (2) centered on the knee head side to the outer side of the thumb and the outer side of the little finger of the metatarsal in the region B1 (1) such that the longitudinal direction of the region B3 was aligned with the weft direction of the obtained woven fabric structure. Specifically, the region B3 was connected to the regions B1 and B2 by hook-and-loop fastener ("Magic Tape" (registered trademark) TMSH-1005W manufactured by Trusco Corporation) on the B1 side and sewing by a sewing machine on the B2 side. The connection length was adjusted such that the angle between the directions of the foot and the body was 90° while force was released, and the region B2 was adjusted such that the knee portion was covered at an angle suitable for walking. In the region B1, the portion other than the instep portion of the foot, and in the region B2, the portion other than the popliteal region were connected to the region A by sewing with a sewing machine, and in each of the regions B1 and B2, the circumferential end portion was bonded by a hook-and-loop fastener, thereby allowing adjusting a desired fastening force.

As a result of performing a wearing test on the produced orthotic, the orthotic force was 5, the feeling of compression was 5, and the rubbing was 5, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot. In addition, the sock shape facilitated the wearing and removing, and facilitated position adjustment of the region B3.

### [Example 2]

A sock type orthotic that was the same as in Example 1 was used, except that both ends of the region B3 were connected to the region B1 and the region B2 by sewing with a sewing machine.

As a result of performing a wearing test on the produced orthotic, the orthotic force was 5, the feeling of compression was 5, and the rubbing was 5, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot. In addition, the position and size of the region B3 were previously adjusted, and therefore wearing and removing were easy.

### [Example 3]

A sock type orthotic that was the same as in Example 1 was used, except that the region B3 was connected to the region A at the ankle front side by sewing with a sewing machine.

As a result of performing a wearing test on the produced orthotic, the orthotic force was 5, the feeling of compression was 5, and the rubbing was 5, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot. In addition, wearing and removing were easy, the region B3 was fitted to the foot, and the wearing appearance was further improved.

### [Example 4]

A sock type orthotic that was the same as in Example 1 was used, except that the region B3 was connected to the region B1 and the region B2 so as to cross at the front side of the foot.

As a result of performing a wearing test on the produced orthotic, the orthotic force was 5, the feeling of compression was 5, and the rubbing was 5, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot. In addition, wearing and removing were easy.

### [Example 5]

A sock type orthotic that was the same as in Example 4 was used, except that the region B3 was connected to the region A at the ankle front side by sewing with a sewing machine.

As a result of performing a wearing test on the produced orthotic, the orthotic force was 5, the feeling of compression was 5, and the rubbing was 5, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot. In addition, wearing and removing were easy, the region B3 was fitted to the foot, and the wearing appearance was further improved.

### [Example 6]

An orthotic for a short limb was produced by using the same fabric as in Example 1 for not the region A but the region B. The region B1 (1) covered the periphery of the metatarsal such that the width direction of the foot was aligned with the weft direction of the obtained woven fabric structure; the region B2 (2) covered the periphery of the knee including the popliteal fossa such that the periphery direction of the knee was aligned with the weft direction of the obtained woven fabric structure; the region B3 (3) connected from both side surfaces of the region B2 (2) centered on the knee head side to the outside of the thumb and the outside of the little finger of the metatarsal of the region B1 (1) such that the longitudinal direction of the region B3 was aligned with the weft direction of the obtained woven fabric structure; and the connection length was adjusted such that the angle between the directions of the foot and the body was 90° while force was released, and the region B2 was adjusted such that the knee portion was covered at an angle suitable for walking. In each of the regions B1 and B2, the periphery end portions were bonded by a hook-and-loop fastener, whereby a desired fastening force was able to be adjusted.

As a result of performing a wearing test on the produced orthotic, the orthotic force was 5, the feeling of compression was 5, and the rubbing was 3, showing the compatibility of the holding force and the orthotic force necessary for the improvement of the deformation and function of the body, with the trackability to friction, fluctuation, compression, and the like occurring during the operation between the orthotic site and the orthotic. In addition, the recovery after deformation was sufficient, and the orthotic was able to be continuously used, exhibiting excellent performance. In addition, when the shoe was worn with the orthotic worn, the shoe was able to be fitted to the size of the foot.

### [Comparative Example 1]

A sock type orthotic that was the same as in Example 1 was used, except that a commercially available rubber band was used for the region B; the region B1 (1) covered the periphery of the metatarsal such that the width direction of the foot was aligned with the warp direction of the rubber band; the region B2 (2) covered the periphery of the knee including the popliteal fossa such that the periphery direction of the knee was aligned with the warp direction of the rubber band; and the region B3 (3) was connected from both side surfaces of the region B2 (2) centered on the knee head side to the outside of the thumb and the outside of the little finger of the metatarsal of the region B1 (1) such that the longitudinal direction of the region B3 was aligned with the weft direction of the rubber band. The commercially available rubber band had an extension ratio of 70% for warp direction and 25% for weft direction under a load of 100 N/5 cm, extension recovery ratio of 95% for warp direction and 80% for weft direction, and a spring constant of 5 N/mm.

As a result of a wearing test for the produced orthotic, the orthotic force was 1, the feeling of compression was 5, and the rubbing was 5; the extension ratio at a load of 100 N/5 cm in the region B was excessively large as the orthotic; and the holding force and the orthotic force necessary for improvement of the deformation and function of the body were insufficient, which were unsuitable.

### [Comparative Example 2]

There was used a shoe horn type orthosis for a short limb, produced by using a polypropylene sheet having a thickness of 3 mm as an orthotic. The polypropylene sheet had an extension ratio of 0.5% by warp and 0.5% by weft under a load of 100 N/5 cm, an extension recovery ratio of 30% by warp and 30% by weft, and a spring constant of 150 N/mm.

As a result of a wearing test for the used orthotic, the orthotic force was 3, the feeling of compression was 3, and the rubbing was 1; the holding force and the orthotic force necessary for improvement of the deformation and function of the body were excessively strong as the orthotic; and there was pain due to the friction occurring during operation between the orthotic site and the orthotic. In addition, when the propylene sheet was deformed, the sheet was not restored to the shape before deformation, and was insufficient for continuous use and thus unsuitable.

### [Comparative Example 3]

There was used a double-sided strut-type orthosis for a short limb with a strut of a stainless steel plate having a thickness of 3 mm as an orthotic. For the stainless steel plate, the extension ratio at a load of 100 N/5 cm was 0% for the warp direction and 0% for the weft direction, and the extension recovery ratio and the spring constant were unmeasurable under the above measurement conditions.

As a result of a wearing test for the produced orthotic, the orthotic force was 3, the feeling of compression was 1, and the rubbing was 1; the holding force and the orthotic force necessary for improvement of the deformation and function of the body were excessively strong as the orthotic; and there was pain due to the friction occurring during operation between the orthotic site and the orthotic, which were unsuitable.

**[Table 1]**

| | Physical properties (region B) | | | | | Orthotic force | Feeling of compression | Rubbing |
|---|---|---|---|---|---|---|---|---|
| | Extension ratio (%) at load of 100 N/5 cm | | Extension recovery ratio (%) | | Spring constant (N/mm) | | | |
| | Warp | Weft | Warp | Weft | | | | |
| Example 1 | 5 | 15 | 85 | 95 | 50 | 5 | 5 | 5 |
| Example 2 | 5 | 15 | 85 | 95 | 50 | 5 | 5 | 5 |
| Example 3 | 5 | 15 | 85 | 95 | 50 | 5 | 5 | 5 |
| Example 4 | 5 | 15 | 85 | 95 | 50 | 5 | 5 | 5 |
| Example 5 | 5 | 15 | 85 | 95 | 50 | 5 | 5 | 5 |
| Example 6 | 5 | 15 | 85 | 95 | 50 | 5 | 5 | 3 |
| Comparative Example 1 | 70 | 25 | 95 | 80 | 5 | 1 | 5 | 5 |
| Comparative Example 2 | 0.5 | 0.5 | 30 | 30 | 150 | 3 | 3 | 1 |
| Comparative Example 3 | 0 | 0 | - | - | - | 3 | 1 | 1 |

### DESCRIPTION OF REFERENCE SIGNS

1: Region B1
2: Region B2
3: Region B3
4: Region A

## Claims

1. An orthotic, comprising: a region B that exhibits an orthotic force when the orthotic is worn; the region B including a region B1 (1) that is configured to cover an orthotic site, a region B2 (2) that is a support region for applying an orthotic force to the orthotic site, and a region B3 (3) that connects the region B1 (1) and the region B2 (2); a structure used for the region B being a woven fabric structure having elastic properties;
**characterized in that** the woven fabric structure is a plain woven fabric composed of inelastic fibers with one of the warp and the weft having a crimp ratio, measured in accordance with the method of JIS L1096 8.7b, of 10% or more and 30% or less and elastic fibers with at least a part of the other having a crimp ratio of 0% or more and 5% or less, and wherein the elastic fibers include polyester elastomer fibers, and the woven fabric structure has an extension ratio at a load of 100 N/5 cm, measured in accordance with the strip method, method A, of JIS L 1096: 2010 8.14.1 JIS method, being 1.0% or more and 20% or less in a warp direction and a weft direction or any one of these directions, and wherein a spring constant, measured according to the method for measuring the spring constant defined in the description, of the woven fabric structure used in the region B1 (1) and the region B3 (3) is 10 N/mm or more and 100 N/mm or less.

2. The orthotic according to claim 1, further comprising a region A (4) that is configured to cover a periphery of the orthotic site, wherein the region A (4) includes at least one or more of structures having elastic properties.

3. The orthotic according to claim 1 or 2, wherein an extension recovery ratio, measured with reference to the strip method, method A, of JIS method, JIS L 1096: 2010 8.15.1, of the woven fabric structure used in the region B after 50 cycle extension under a load of 100 N/5 cm is 80% to 100% in the warp direction and the weft direction or any one of these directions.

4. The orthotic according to any one of claims 1 to 3, wherein the region B1 (1) and/or the region B2 (2) comprises a mechanism capable of adjusting fastening force during wearing.

5. The orthotic according to any one of claims 1 to 4, comprising a mechanism that is openable and closable during wearing.

6. The orthotic according to any one of claims 1 to 5, wherein the region A (4) and the region B are integrally formed by being connected to each other.

7. The orthotic according to any one of claims 1 to 6, being an upper limb orthosis.

8. The orthotic according to any one of claims 1 to 6, being a trunk orthosis.

9. The orthotic according to any one of claims 1 to 6, being a lower limb orthosis.

## Patentansprüche

1. Orthese, die Folgendes umfasst: eine Region B, die eine orthetische Kraft ausübt, wenn die Orthese getragen wird; wobei die Region B eine Region B1 (1), die zum Bedecken einer orthetischen Stelle ausgelegt ist, eine Region B2 (2), die eine Stützregion zum Anwenden einer orthetischen Kraft auf die orthetische Stelle ist, und eine Region B3 (3), welche die Region B1 (1) und die Region B2 (2) verbindet, umfasst; eine Struktur, die für die Region B verwendet wird, die eine Gewebestruktur mit elastischen Eigenschaften ist;
**dadurch gekennzeichnet, dass** die Gewebestruktur ein Leinwandgewebe ist, das aus nicht elastischen Fasern, wobei eines aus Kette und Schuss ein gemäß dem Verfahren von JIS L1096 8.7b gemessenes Kräuselungsverhältnis von 10 % oder mehr und 30 % oder weniger aufweist, und elastischen Fasern, wobei zumindest ein Teil des anderen ein Kräuselungsverhältnis von 0 % oder mehr und 5 % oder weniger aufweist, besteht und wobei die elastischen Fasern Polyester-Elastomerfasern umfassen und die Gewebestruktur ein gemäß dem Streifenverfahren, Verfahren A, von JIS L 1096: 2010 8.14.1. JIS-Verfahren gemessenes Dehnungsverhältnis bei einer Belastung von 100 N/5 cm von 1,0 % oder mehr und 20 % oder weniger in Kettrichtung und in Schussrichtung oder in einer dieser Richtungen aufweist und wobei die gemäß dem in der Beschreibung definierten Verfahren zum Messen der Federkonstante gemessene Federkonstante der in Region B1 (1) und in Region B3 (3) verwendeten Gewebestruktur 10 N/mm oder mehr und 100 /N/mm oder weniger beträgt.

2. Orthese nach Anspruch 1, die weiters eine Region A (4) umfasst, die dazu ausgelegt ist, den Umfang der orthetischen Stelle zu bedecken, wobei die Region A (4) zumindest eine oder mehrere aus Strukturen mit elastischen Eigenschaften umfasst.

3. Orthese nach Anspruch 1 oder 2, wobei ein in Bezug auf das Streifenverfahren, Verfahren A, des JIS-Verfahrens JIS L 1096: 2010 8.15.1. gemessenes Dehnungsrückstellungsverhältnis der in Region B verwendeten Gewebestruktur nach Dehnung über 50 Zyklen unter einer Belastung von 100 N/5 cm 80 % bis 100 % in Kettrichtung und in Schussrichtung oder in einer dieser Richtungen beträgt.

4. Orthese nach einem der Ansprüche 1 bis 3, wobei die Region B1 (1) und/oder die Region B2 (2) einen Mechanismus umfassen/umfasst, der in der Lage ist, die Befestigungskraft während des Tragens einzustellen.

5. Orthese nach einem der Ansprüche 1 bis 4, die einen Mechanismus umfasst, der während des Tragens zu öffnen und zu schließen ist.

6. Orthese nach einem der Ansprüche 1 bis 5, wobei die Region A (4) und die Region B einstückig ausgebildet sind, indem sie miteinander verbunden sind.

7. Orthese nach einem der Ansprüche 1 bis 6, die eine Orthese für die oberen Extremitäten ist.

8. Orthese nach einem der Ansprüche 1 bis 6, die eine Rumpforthese ist.

9. Orthese nach einem der Ansprüche 1 bis 6, die eine Orthese für die unteren Extremitäten ist.

## Revendications

1. Orthèse, comprenant : une région B qui présente une force orthétique lorsque l'orthèse est portée ; la région B comprenant une région B1 (1) qui est configurée pour couvrir un site orthétique, une région B2 (2) qui est une région de support pour appliquer une force orthétique au site orthétique, et une région B3 (3) qui relie la région B1 (1) et la région B2 (2) ; une structure utilisée pour la région B étant une structure de tissu tissé présentant des propriétés élastiques ;
**caractérisée en ce que** la structure de tissu tissé est un tissu tissé uni composé de fibres inélastiques avec une certaine de la chaîne et de la trame présentant un taux de frisure, mesuré selon le procédé de JIS L1096 8.7b, de 10 % ou plus et de 30 % ou moins et de fibres élastiques avec au moins une partie de l'autre présentant un taux de frisure de 0 % ou plus et de 5 % ou moins, et dans laquelle les fibres élastiques comprennent des fibres d'élastomère de polyester, et la structure de tissu tissé présente un taux d'extension à une charge de 100 N/5 cm, mesuré selon le procédé de bande, procédé A, du procédé JIS de JIS L 1096 : 2010 8.14.1, étant 1,0 % ou plus et 20 % ou moins dans une direction de chaîne et une direction de trame ou l'une quelconque de ces directions, et dans laquelle une constante de ressort, mesurée selon le procédé de mesure de la constante de ressort défini dans la description, de la structure de tissu tissé utilisée dans la région B1 (1) et la région B3 (3) est de 10 N/mm ou plus et de 100 N/mm ou moins.

2. Orthèse selon la revendication 1, comprenant en outre une région A (4) qui est configurée pour couvrir une périphérie du site orthétique, dans laquelle la région A (4) comprend au moins une ou plusieurs des structures présentant des propriétés élastiques.

3. Orthèse selon la revendication 1 ou 2, dans laquelle un taux de récupération d'extension, mesuré par rapport au procédé de bande, procédé A, du procédé JIS, JIS L 1096 : 2010 8.15.1, de la structure de tissu tissé utilisée dans la région B après une extension de 50 cycles sous une charge de 100 N/5 cm est de 80 % à 100 % dans la direction de chaîne et la direction de trame ou l'une quelconque de ces directions.

4. Orthèse selon l'une quelconque des revendications 1 à 3, dans laquelle la région B1 (1) et/ou la région B2 (2) comprend un mécanisme capable d'ajuster la force de fixation en utilisation.

5. Orthèse selon l'une quelconque des revendications 1 à 4, comprenant un mécanisme qui peut être ouvert et fermé en utilisation.

6. Orthèse selon l'une quelconque des revendications 1 à 5, dans laquelle la région A (4) et la région B sont formées d'un seul tenant en étant connectées l'une à l'autre.

7. Orthèse selon l'une quelconque des revendications 1 à 6, étant une orthèse de membre supérieur.

8. Orthèse selon l'une quelconque des revendications 1 à 6, étant une orthèse de tronc.

9. Orthèse selon l'une quelconque des revendications 1 à 6, étant une orthèse de membre inférieur.
